# EUROPEAN PATENT APPLICATION

(11) **EP 2 767 585 A1**
(43) Date of publication of application: **20.08.2014**
(21) Application number: 13168886.3
(22) Date of filing: 23.05.2013
(51) Int. Cl.: C12M 1/00, B01D 53/52, B01D 53/84

(54) **Microbiological method of H2S removal from biogas**

(30) Priority: 18.02.2013 EP 13461504
(71) Applicant: Politechnika Lódzka, 90-924 Lódz (PL)
(72) Inventor: Zieminski, Krzysztof, 93-355 Lódz (PL); Cwalina, Beata, 41-106 Siemianowice Sl. (PL); Kopycki, Wlodzimierz, 91-214 Lódz (PL)
(74) Representative: Witek, Rafal

(57) **Abstract**

The present invention is based on a method of purification of a microbiological biogas arising from the methane fermentation of settled organic matter in a fermentation container, based on the removal of contaminants, particularly H₂S, under anoxic conditions, in an installation containing a biofilter loaded with a biological filter bed sprinkled with a mineral medium, containing immobilised microorganisms capable of degrading sulphur compounds, characterised in that the biological filter bed is sprinkled with a medium in the form of a solution containing nitrogenous salts of alkali metals of the I and II group, wherein the concentration of nitrogen ions in the medium is contained in the range of 20 to 2500 mg/l, preferably in the range of 50 to 1000 mg/L, whereas the concentration of H₂S loaded into the installation is 100 - 3000 ppm.

## Description

The subject of the present invention is a method of eliminating contaminants from biogas, particularly sulphur compounds, conducted in the presence of nitrogen oxides in a biofilter containing a biological filter bed. The present invention is part of the science of biotechnical methods for the purification of biogas obtained from various sources. Biogas is a mixture of fermentation gasses arising from the activity of anaerobic bacteria resulting in the degradation of organic substances. This is a substrate obtained from the degradation of organic substances by methanogenic anaerobic bacteria, characterised by its varying composition which is dependent on many factors which may include: initial composition and form of the organic substance, its moisture, pH, temperature and pressure. The biogas mixture usually contains about 55-65% methane (CH₄), 30-45% carbon dioxide (CO₂), 0.01-5% hydrogen sulphide (H₂S) and low concentrations of nitrogen, oxygen and water vapour. The percentage composition of methane in biogas constitutes the combustible value of this fuel. Two types of biogas are differentiable: landfill biogas which arises spontaneously in waste dumps and production biogas produced via the purposeful fermentation in bioreactors (mainly in wastewater treatment plants or in agricultural biogas installations). Landfill biogas arises as a result of the biochemical conversion of waste containing biodegradable substances. The main components of landfill biogas are: methane, carbon dioxide and nitrogen. In addition, it may also contain: oxygen, hydrogen, ammonia, sulphur compounds, aliphatic hydrocarbons other than methane, aromatic hydrocarbons and chloride derivatives.

Landfill gas may pose numerous dangers attributable to environmental degradation such as acid rain and potentiating of greenhouse gasses. Landfill gas also poses a threat to humans, which is connected with the dispersal of odorants, toxins and groundwater contamination. For the above reasons, it is becoming common to collect landfill gasses. However, its treatment to make a composition capable of being added to the gas supply system is very troublesome and expensive using conventional methods, mainly based on the absorptive properties of activated carbon.

Biogas forms due to the anaerobic fermentation (methane) of an active deposit used in the purification of sewage. Anaerobic stabilization consists of the degradation of organic substances contained in deposits under anaerobic conditions. Anaerobic bacteria degrade organic substances into simple compounds. The end products of biochemical processes therein are gasses, mainly methane and carbon dioxide. H₂S, hydrogen, nitrogen and precipitates are also formed.

One of the methods which may be used in the reclamation of biological-organic waste from agriculture and the food industry is the anaerobic production of biogas. Biogas production from animal wastes in agricultural biogas plants eliminates such pathogens as *Salmonella, Escherichia coli,* tuberculosis, and foot-and-mouth viruses. Additionally, improved field fertilization conditions are achieved in comparison to unfermented dung, as well as the ability to maintain humus equilibrium in soil and the destruction of weed seeds, thereby reducing herbicide use. The transformation and utilization of biogas from animal wastes results in an 80% reduction of odours, reduced risk of groundwater contamination and free greenhouse gas emission, including methane.

An observed need in the state of the art is the increase of the calorific value of the resulting biogas fuel. The difference between the fuel value of methane and biogas shows that the purification of biogas from superfluous components greatly increases its calorific value. The main reason for the decreased worth of biogas as a fuel is the presence of contaminants, particularly H₂S.

H₂S is formed during methane fermentation due to: a) anaerobic decomposition of organic compounds (proteins containing sulphur-containing amino-acids, thioalcohols, thioglycosides, alkaloids), b) dissimilative reduction of sulphates (sulphate respiration), c) reduction of elemental sulphur (sulphur respiration). H₂S is a significant component affecting the costs connected with biogas use, because its concentration may be from 0.1 to 10 g H₂S/m³ (0.01 - 5% by volume). Thus, the most important process in readying biogas for use in burning is sulphur removal.

The need to remove sulphur from biogas also stems from the negative effect of H₂S on biogas combustion devices, the need to protect electrical generators, and the protection of boiler burners and boilers from corrosion. The sulphur present in biogas may cause failures in co-generation systems, which greatly reduces their service life. The need to remove H₂S from biogas stems, amongst other reasons, from its direct participation in the formation of aggressive sulphuric acid. The sulphur-loaded gas corrodes piping, fittings and boiler elements, as well as gas storage vessels, which leads to frequent interruptions and greatly reduces their service life. Due to the combustion of sulphurous biogas, there is an increase in sulphur dioxide emissions, which is a cause of acid rain. During the production and use of biogas, emphasis is placed on technologies that do not burden the environment. When low-purity biogas is combusted, there may be an increase in the emission of sulphur, nitrogen, oxygen and carbon dioxide. Depending on the source of the biogas, it may contain a series of substances which during combustion may contribute to the uncontrolled formation of strongly toxic compounds, such as sulphur dioxide. Thus there is a need to limit the emission of H₂S, a strongly toxic component, that is deleterious to human health and the natural environment.

The remaining percentage of other contaminants comprises ammonia and carbon monoxide as well as organic substances and dust.

In the general paucity of desulphuring methods for biogas, it is possible to base upon methods of desulphuring gaseous fuels designed and optimized for the petrochemical and gas industries. In general, desulphuring methods may be divided into: a) dry, b) wet, c) biological.

Dry methods of desulphuring methods are based mainly on adsorption on solid sorbents, metal oxides or activated carbon. They are characterised by the fact that the chemical process of H₂S binding occurs in a dry state, in a gas-solid system. Another dry technology are desulphuring methods using granulated iron oxide. Therein, due to surface contact with the filter bed, H₂S reacts with iron hydroxide. As a result of this reaction, sulphur binds with the iron to form iron sulphide and water vapour or water is released.

The efficacy of dry methods of desulphuring is mainly dependent on the initial concentration of H₂S fed into the desulphuring system. With an initial H₂S concentration of about 1400 mg/m³ the desulphuring efficiency is about 30%. For H₂S initially at 280 mg/m³, it is reduced by 75%. A solid filter bed desulphurs biogas with decreasing efficiency over time. In the first month, a freshly loaded filter bed cleans biogas from 2000 mg/m³ H₂S to 280 mg/m³. After a month, the same filter bed cleans biogas from 2700 mg/m³ to 1700 mg/m³. The manual removal of the filter bed is also troublesome, both in the case of bog ore and granulated iron oxide. This is compounded by the need to use additives, coagulants, in the purification process, which greatly increases effluent purification costs. There are also difficulties in maintaining the stability of the entire desulphuring process.

Wet methods of desulphuring biogas are based on rinsing biogas with different solutions that absorb H₂S. The development of this method has lead to the design of technologies that may be divided into two groups: a) absorption of H₂S and its elution from the rinsing solution in unchanged form, b) absorption of H₂S and its oxidation to other sulphur forms (into elemental sulphur, in the newest methods). Most commercial H₂S removal technologies are chemical processes. A number of problems are encountered using the above desulphuring methods. This includes the need to maintain moisture in the ore bed, and the need to regenerate/enhance bog ore (desulphuring is more effective in an alkaline environment). The regeneration of bog ore occurs in contact with oxygen under moist conditions, which means that the extracted ore is periodically moved manually to intensify its contact with atmospheric oxygen, while pouring water on the pile. The next problem is the generation of waste, and the need to continually analyze biogas so as to ascertain the moment in which the filter material should be exchanged. Negligence in this respect not only will lead to the accelerated deterioration of equipment for the energy producing use of biogas, but the combustion of poorly purified biogas releases considerable quantities of sulphur oxides into the atmosphere. A system for desulphuring biogas based on iron hydroxide loses its effectiveness over time and with increased H₂S concentrations in biogas directed into the desulphuring filter. On the basis of measurements made to date, it can be concluded that the loss of effectiveness is on average 50% over 6 months. Additionally, the desulphuring system in question causes an increase in the oxygen content of purified biogas, thus reducing the volume concentration of methane therein and thereby also its calorific value.

The drawbacks of using wet methods include extensive investment as well as maintenance costs of the systems, often exceeding potential benefits to be drawn from the energy drawn from co-generation systems, the need to use expensive chemical reagents, large energy expenditures for the reaction as well as the problem caused by the need to reclaim and neutralize waste from purification.

For the above reasons, it is desirable to develop biological methods for the purification of biogas as an alternative to chemical processes. These methods have a large potential to overcome some or all of the drawbacks of chemical methods. Biological biogas purification methods are based on the biodegradation of H₂S, the removal of H₂S from biogas using sulphurous bacteria via biochemical processes in special vessels, bioreactors. They are an attractive option due to low capital outlay as well as no negative environmental effects. The contaminants formed during microbial activity settled on the reactor fill are degraded into harmless products such as water, CO₂ and mineral compounds. The main point of biotechnical methods is to possess appropriate, specially selected microorganisms, as well as to know the conditions under which they may be used.

At present, the key problem is the lack of highly effective methods of biological biogas purification on an industrial scale. Intensive research efforts are currently underway related to the use of biological methods that make it possible to remove H₂S as well as sulphur derivatives. However, we have not been able to find literature which would indicate the industrial scale use of solutions similar to those described herein. Industrial biogas purification conditions differ from laboratory systems and pose additional obstacles in the design of a system facilitating the production of highly pure biogas. This is affected by, for example, variability of the biogas composition, changes in H₂S concentration or shifts in the biodiversity of microorganisms. The biogas production and purification process under industrial conditions is dynamic and the definition of set parameters for the functioning of such a system has proven exceedingly difficult to specialists.

Another problem entailed by the use of biological methods is the presence of elemental sulphur in the gas desulphuring installation. Sulphur compounds are oxidized to elemental sulphur, which is deposited in the system and must be regularly removed. Document EP1604727 discloses a process for desulphuring biogas conducted using halotolerant organisms (i.e. *Thiobacillus),* on a carrier sprayed with an aqueous solution containing iron and sulphate ions. This process is conducted under oxic conditions using such nutritive components as (NH₄)₂SO₄, KC1, K₂HPO₄, MgSO₄, Ca(NO₃)₂ in the medium. A reaction resulting in elemental sulphur occurs in the described purification process.

Document WO2008131034 discloses a method of removing H₂S from biogas arising in an anoxic environment using a sprayed biofilter system. The process makes use of natural or artificial substrates (polyurethane foam), and of H₂S - oxidizing bacteria which were obtained from hot springs, wherein they develop in a pH of 3. The spraying medium is a liquid effluent containing microelements: potassium, phosphorus and iron. H₂S is oxidized to sulphur and SO4²⁻.

Publication WO05037403 discloses a biofilter containing a designed filler, on which bacteria selected from the group of *Thiobacillus thioparus, begigiatoa, thopthrix genera, feroxidants* have been immobilized. One of the end products of purification is elemental sulphur.

Thus, from the state of the art stems a need to design a system overcoming current obstacles.

The goal of the present invention is a 97-100% reduction of H₂S conducted under industrial conditions, which will make it possible to attain parameters akin to natural gas. The goal of the present invention is the selection of the optimal conditions for the process, ensuring highly effective desulphuring on an industrial scale. The next goal of the present invention is the minimalisation of sulphur production in biogas purification and of deposits formed in the installation.

Unexpectedly, this goal has been achieved by the present invention.

The present invention is a method for the microbiological purification of biogas arising from the methane fermentation of organic deposits in a fermentation tank based on the removal of contaminants, particularly H₂S, in anoxic conditions, on an installation containing a biofilter loaded with a filter bed sprayed with a mineral medium, on which hydrogen sulphide degrading microorganisms have been immobilized, characterised in that the biological filter bed is sprinkled with a medium in the form of a solution nitrous salts of alkali metals of the I and II group, wherein the concentration of nitrogen ions in the medium is contained in the range of 20 to 2500 mg/1, preferably in the range of 50 to 1000 mg/L, whereas the concentration of purified H₂S is 100 - 3000 ppm.

Preferably, the concentration of nitrogen in the medium is contained in the range of 50 - 200 mg/L, wherein the concentration of loaded H₂S into the installation is 300 ppm.

Preferably, the concentration of nitrogen in the medium is contained in the range of 260-800 mg/L, wherein the concentration of H₂S loaded into the installation is 720 ppm. Preferably, the medium flow volume intensity sprinkled onto the biological filter bed is contained in the range of 5 L/h to 500 L/h per cubic metre of filter bed.

Preferably, the S/N value is from 1 to 30, more preferably 2 to 20.

Preferably, the process temperature is 10 to 35°C, preferably 20-30 °C.

Preferably, the medium pH is 5-8, preferably no less than 5.5 and no more than 7.0-7.5. The biological purification of gasses is realized mainly in the following types of installations: biofilter, bioscrubber and biofilter with a sprinkled layer, called a three-phase bioreactor. These systems are composed of three phases: solid (fill), liquid and gas. Processes conducted therein also occur according to the same mechanism. Gaseous contaminants are absorbed and brought into the liquid phase, and are then degraded by microbes into inorganic compounds such as water and mineral salts.

In bioscrubbers, contaminants from exhaust gasses are absorbed into a liquid, in which microorganisms are dispersed, whereas the biological regeneration of the liquid occurs in a settlement tank. Absorption and regeneration thus proceeds in two separate compartments. This system is composed of a column, in which gaseous contaminants are absorbed by a liquid which is then fed into the bioreactor.

Nishimura and Yoda *(*Nashimura S., and Yoda M., 1997. Removal of hydrogen sulphide from anaerobie biogas using a bio-scrubber. Water Science and Technology 36 (6-7): 349-356*)* successfully used a bioscrubber purify biogas from anaerobic fermentation. The bioreactor was supplied with air and settlement from sewage treatment. 99% of H₂S was oxidized to sulphates.

Based on a bioscrubber, the Dutch company PAQUES has designed the TIOPAQ industrial biogas desulphuring technology. This method is very effective, ensures the reduction of sulphur compounds contained in biogas by 99%. Sulphur compounds are mainly oxidized to elemental sulphur, which must then be removed from the system.

In a biofilter, the main element of the biological filter is a porous filter material layer colonized by microorganisms. The carrier is often a biological material (bark, peat, compost, sewage solids) containing nutrients. In the biofilter, the premoistened efflux gasses are passed through solid biological beds in which they are degraded by the immobilized microorganisms. During the slow throughput of gasses through the filter material, the contaminants are absorbed, and then degraded by the microorganisms. The microorganism activity regenerates the filter bed, the absorbent. In a biofilter, different than in bioscrubbers, the absorption and degradation of contaminants occur in the same location in the device. The drawback of bio filters is filter bed dishomogeneity that may periodically occur, acidification (H₂SO₄, HC1) and filter bed compaction.

In triphase bioreactors, microorganisms are settled onto a solid fill that is neutral to microorganisms (Raschiga rings, glass spheres, ceramics, plastics or other synthetic materials). The liquid phase (water with nutritive salts) flows in a thin film along the film, dampening the biological layer. The gas flows concurrently with the liquid. The liquid-absorbed contaminants diffuse to the biological layer (biofilm on the fill surface) and are biodegraded. Absorption and regeneration of contaminants occur in the same unit.

When comparing methods of biodegradation, attention should be paid to the fact that the rate of elimination of contaminants in a three-phase bioreactor is higher than in a bioscrubber, with a lower energy use. One should also note the very good control of the contaminant biodegradation process conducted in three-phase bioreactors. Bacteria develop in the bioreactor (so-called counter-current rinse), which acts as an absorbent, and has a synthetic fill. This filter bed makes it possible to localize and proliferate bacteria. To ensure the correct conditions for bacterial development, it is necessary to use additional devices to augment the process, mainly to ensure sufficient oxygen. Oxygen is pumped into the biogas pipeline ahead of the biological reactor. The amount of oxygen is selected automatically and proportionally to the biogas flow. At the same time, the oxygen level in the biogas is measured at the exit from the reactor. The medium for the microorganisms consists of the water from a gravity settlement tank. The present invention was embodied using a system based on a sprinkled biofilter. A sprinkled biofilter is one filled with a synthetic or ceramic fill, or with activated carbon. Contaminated gas is fed in from the bottom of the device and recirculated water containing nutrients is sprinkled onto the fill from the top. Microorganisms are immobilised on the carrier. An example of the industrial use of a sprinkled biofilter in the microbiological desulphuring is the MICROBIAL system. This system is based on the activity of sulphur bacteria in oxic conditions, which are sprinkled with initially treated sewage.

Bacteria desirable in the biodegradation of H₂S should have: a) the ability to efficiently oxidize H₂S and b) minimal nutritive requirements. In recent years, research has been performed on photoautotrophic as well as chemotrophic bacteria.

### Selected types of chemotrophs and further possible types of H₂S degradation

| Bacterial genus | Electron donor | Electron acceptor | Carbon source | Products |
|---|---|---|---|---|
| *Thiobacillus sp.* (in general) | S⁰, H₂S, S₂O₃²⁻ - | O₂ | CO₂ | SO₄²⁻ |
| *Thiobacillus denitrificans* | S⁰, H₂S, S₂O₃²⁻ | O₂, NO³⁻ | CO₂ | SO₄²⁻, N₂ |
| *Thiobacillus ferrooxidans* | Fe²⁺, S⁰, H₂S | O₂ | CO₂ | F2³⁺, SO₄²⁻ |

From the point of view of biogas purification, the most significant example of *Thiobacillus* is *T. denitrificans.* This is a widespread, well characterised obligatory chemoautolithotroph, with unusual biochemical characteristics. *Thiobacillus denitrificans* is the best known species possessing the ability to oxidize inorganic sulphur compounds (H₂S, thiosulphate). Additionally under anoxic conditions, it has the ability to reduce nitrogen to N₂. It differs from many other known oxidizing-oxidizing chemolithotrophic such as *Acidiothiobacillus ferrooxidans* in that it is a facultative aerobe and may respire oxically or through denitrification. Furthermore, in contrast to obligate chemolithotrophic anaerobes, it does not acidify the environment and prefers a neutral pH. Due to their properties, these bacteria are particularly important for environmental bioremediation.

Microorganisms used in the present invention constitute a microbial consortium isolated from natural environments rich in sulphur. They form a specific group capable of purifying biogas.

Experiments according to the present invention were performed in a biofilter working as part of an industrial installation. The process is conducted in a biofilter loaded with various types of fill (natural and synthetic). These beds were inoculated with a group of bacteria collected from natural environments rich in sulphur. Microbes that degrade the contaminants are immobilized on the fill material. They form a specific system, a biofilm, characterized by its highly efficient degradation of H₂S. The use of nitrogen in the process eliminates the stage of aerating the biogas as well as greatly easing the biogas purification process. In effect, the technology proposed as part of this project is very competitive in relation to extant solutions.

The process is conducted in anoxic conditions, and the purified biogas is not diluted with oxygen. This is significant, because it makes it possible to avoid the dangerous formation of an explosive oxygen-biogas mixture. The lack of the need to aerate also decreases the maintenance costs of the installation. Systems present on the market require oxygen supply.

The process according to the present invention does not result in the deposition of elemental sulphur nor in decreased pH, which is greatly advantageous, because there is no need to remove excess sulphur from the system, nor to correct the pH. Most sulphur bacteria develop at a low pH, whereas the embodied process runs at a neutral pH and requires no correction.

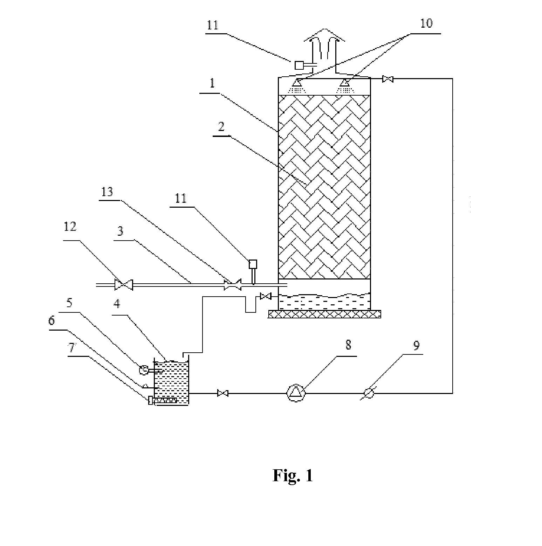

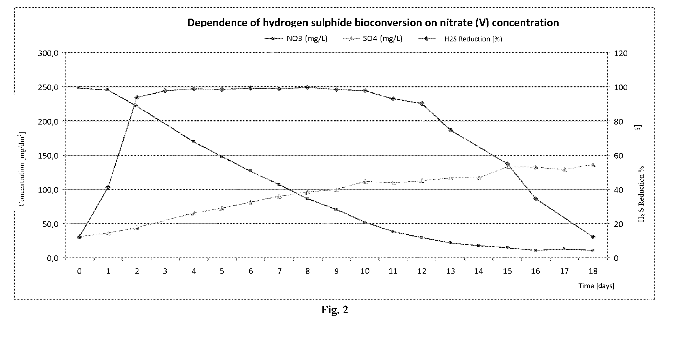

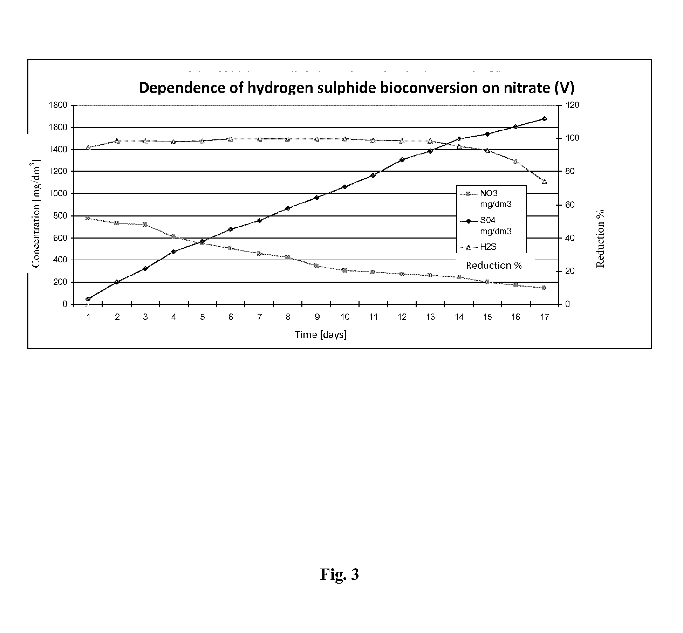

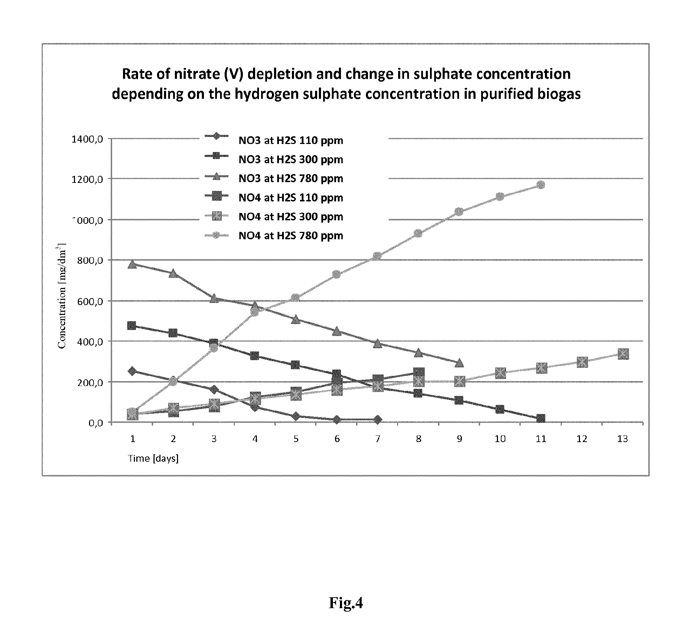

The present invention is described in detail in the example embodiments in the figures, where Fig. 1 represents an biogas purification installation consisting of a biofilter filled with a carrier. Contaminated gas is fed in through the bottom of the device, and the recirculated water containing nutrients is sprinkled from the top. Microorganisms are immobilized on the carrier. Fig. 2 represents the effect of the concentration of nitrogen, one of the components of medium for the reduction of H₂S as well as for the process of sulphide ion formation instead of elemental sulphur. The concentration of H₂S in the output purified biogas is 300 ppm. The graph represents changes in sulphide concentration over time. It is clearly visible that the process occurs with sulphide formation and their concentration grew continuously. Fig 3 represents the effect of nitrogen concentration on H₂S reduction, as well as the process of sulphate ion formation instead of elemental sulphur. The concentration of H₂S supplied to the biogas purification is 720 ppm. Fig. 4 represents the rate of nitrogen depletion (V) as well as changes in sulphate concentration depending on the concentration of hydrogen sulphide in the biogas being desulphured.

### Example 1

Biogas formed during methane fermentation is directed with a pressure of 3-5 kPa to a pipeline (3) and thence to a system for biological desulphuring. The amount of gas flow is regulated using a flow regulator (13). H₂S concentration measurement and recording at the input and output from the biofilter is performed using H₂S concentration sensors (11). The biological filter bed, being a porous filter material (2) that facilitates the formation of a specific biofilm exhibiting a biogas purification securing activity and is found in the biofilter column (1). To ensure optimal conditions for the development and selection of microbes, the filter bed is sprinkled from a sprinkler (10) at the top of the biofilter with a specific medium containing nitrogen at an appropriate concentration. The medium percolating through the filter bed to the bottom of the column is carried off to a container (4). From there, the medium is returned using a pump (8) through a medium flow meter (9) to the sprinkler (10). The medium holding container (4) possesses a heater (7) along with a temperature sensor (5) which facilitates the maintenance of a constant temperature, as well as a medium level sensor (6). The biogas supply to the installation is cut off with a valve (12).

The experiment was performed using an installation input H₂S concentration of 300 ppm. The concentration of nitrogen in the medium is contained in the range of 200 mg/L - 50 mg/L.

The reduction of H₂S from biogas by 97-100 % is attained with a nitrogen concentration in the medium no lower than 50 mg/L. This dependence occurs when the concentration of H₂S in the biogas does not exceed 500 mg/dm³ (Fig. 2). Lower concentrations of nitrogen do not ensure such high levels of H₂S reduction. For example, a 90,1% reduction of H₂S was attained with a medium nitrogen concentration of 29.3 mg/dm³, and a 54.8% H₂S reduction with a medium nitrogen concentration of 14.4 mg/dm³.

Fig. 3 shows the dependence of the effect of biogas desulphuring on the medium nitrogen concentration. The desulphuring system was supplied with a biogas containing 720 ppm hydrogen sulphide. The data shown in Fig. 3 show that a high, in excess of 99%, hydrogen sulphide reduction was attained when the concentration of nitrogen in the medium was maintained at a level from 800 mg/dm³ to about 260 mg/dm³. Below this value, we noted a deterioration of the reduction of hydrogen sulphide and with a medium nitrogen concentration of 146 mg/dm³ we only attained a 73% reduction of hydrogen sulphide.

During the study performed, we also noted that hydrogen undergoes bioconversion to sulphates. Their concentration in the medium sprinkled onto the biological filter bed is dependent on the amount of hydrogen sulphide in the supplied biogas. It was shown that a concentration of 1600 mg/dm³ of sulphates in the medium does not negatively affect the process.

When we subjected biogas containing 780 ppm hydrogen sulphide (about 1092 mg H₂S/dm³) to purification, over 9 days we observed a decrease in nitrogen concentration from a value of 778.7 mg/dm³ to 295.1 mg/dm³, a reduction by 62.1 % (Fig.4).

At the same time the sulphide concentration grew from an initial value of 51.1 mg/dm3 to 1167 mg/dm³, and this value did not adversely affect the desulphuring process.

When purifying a biogas with a hydrogen sulphide concentration of 300 ppm (about 420 mg/dm³), it was determined after 11 days that the assimilation of nitrogen of about 96%, the concentration of this anion decreased from 474.5 mg/dm³ to 14.6 mg/dm³. The increase in sulphide concentration was in this case decidedly slower in comparison to purification of biogas containing hydrogen sulphide at 780 ppm. The value of this anion (SO₄⁻) grew from 51.1 mg/dm³ to 337.4 mg/dm³. A similar character of changes in nitrogen (V) and sulphide concentration was observed during purification of biogas containing hydrogen sulphide at a concentration of 110 ppm.

Biogas is a renewable energy, environmentally friendly and easily available. Biogas purification technology is a solution essential to the development of an energy industry based on biogas, because it makes it possible to remove contaminants, which at present make the mass use of biogas as an ecological fuel impossible. The resulting, purified biogas is suitable for a wide array of uses. It may be used for heating energy, electrical energy, heat and electricity generation in combined systems, vehicle fuel, in methane production, or else fed into the gas supply system.

The limitation of elemental sulphur production by the conversion of contaminants to sulphides prevents the formation of residues, and eliminates the need to remove them from the system, which simplifies the process and increases its effectiveness.

## Claims

1. A method of microbiological purification of biogas arising during the methane fermentation of settled organic matter in a fermentation container, based on the removal contaminants, particularly H₂S, under anoxic conditions, in an installation containing a biofilter loaded with a biological filter bed sprinkled with a mineral medium, containing immobilised microorganisms capable of degrading hydrogen sulphide, **characterised in that** the biological filter bed is sprinkled with a medium in the form of a solution containing nitrogenous salts of alkali metals of the I and II group, wherein the concentration of nitrogen ions in the medium is contained in the range from 20 to 2500 mg/l, preferably in the range of 50 to 1000 mg/L, whereas the concentration of H₂S loaded into the installation is 100 - 3000 ppm.

2. A method according to Claim 1, **characterised in that** the concentration of nitrogen in the medium is contained in the range of 50 - 200 mg/L, wherein the concentration of H₂S loaded into the installation is 300 ppm.

3. A method according to Claim 1, **characterised in that** the concentration of nitrogen in the medium is contained in the range of 260 - 800 mg/L, wherein the concentration of H₂S loaded into the installation is 720 ppm.

4. A method according to Claim 1, **characterised in that** the medium flow volume intensity sprinkled onto the biological filter bed is contained in the range of 5 L/h to 500 L/h per cubic metre of filter bed.

5. A method according to Claim 1, **characterised in that** the S/N value is from 1 to 30, preferably 2 to 20.

6. A method according to any of the above Claims, **characterised in that** the process temperature is from 10 to 35°C, preferably 20-30 °C.

7. A method according to any of the above Claims, **characterised in that** the medium pH is 5-8, preferably no less than 5.5 and no more than 7.0.
